# EUROPEAN PATENT APPLICATION

(11) **EP 3 988 062 A1**
(43) Date of publication of application: **27.04.2022**
(21) Application number: 19933395.6
(22) Date of filing: 19.06.2019
(51) Int. Cl.: A61F 2/90

(54) **SEGMENTAL COVERED STENT AND PREPARATION METHOD THEREFOR**

(71) Applicant: Hangzhou Endonom Medtech Co., Ltd, Binjiang District Hangzhou Zhejiang 310052 (CN)
(72) Inventor: WANG, Yongsheng, Hangzhou, Zhejiang 310052 (CN); LI, Anwei, Hangzhou, Zhejiang 310052 (CN)
(74) Representative: Petraz, Gilberto Luigi
(86) International application number: PCT/CN2019/091935
(87) International publication number: WO 2020/252702

(57) **Abstract**

Provided is a segmented covered stent (100), which includes a covering membrane (120) and a support frame (110) fixed to the covering membrane (120). The support frame (110) comprises an annular structure (1200) and a spiral structure (1300), wherein the annular structure (1200) is formed by a plurality of first wave-shaped units (1100) connected end to end; and the spiral structure (1300) is a tubular structure formed by a plurality of second wave-shaped units (1400) connected end to end and arranged in a continuous spiral manner, and the overall extension direction of the spiral structure (1300) is parallel to the support frame (110). A method for preparing the segmented covered stent (100) is further provided. In the segmented covered stent (100), the annular structure (1200) provides a good radial support force to reduce the compression force, the spiral structure (1300) has good flexibility to adapt to the shape change for various curved and arch-shaped branch vessels, and the annular structure (1200) and the spiral structure (1300) are combined to adapt to different requirements of different branch vessels regarding the flexibility of the covered stent.

## Description

### TECHNICAL FIELD

The present invention relates to the technical field of medical devices, and particularly to a segmented covered stent and a preparation method therefor.

### BACKGROUND

The middle layer of the artery wall of normal human blood vessels is rich in elastic fibers, and can dilate and contract with the heartbeat to deliver blood. If the middle layer of the artery wall is damaged, the elastic fiber breaks and fibrous scar tissue is formed instead. As a result, the artery wall loses its elasticity, and cannot withstand the impact of blood flow, which causes the bulging of blood vessel walls or tearing of intima, forming an aortic aneurysm or dissection. Aortic dissection is a life-threatening aortic disease with rapid progress and high mortality, wherein the incidence is about 0.3%, the early mortality without treatment increases by 1% per hour, and more than 50% of patients will die within one week.

With the development of medical technologies, more and more surgical methods such as open surgery, hybrid surgery or minimally invasive surgery are adopted for the active treatment of life-threatening aortic diseases. Endovascular aortic repair is such a technology that a covered stent is placed at the lesion site by a minimally invasive method to isolate the lesion, and effectively protect the aortic aneurysm wall from the compression of systemic arterial pressure, thus preventing the aortic aneurysm and dissection rupture.

By virtue of small trauma and definite treatment effect, endovascular aortic repair has become an important option in the treatment of type B aortic dissection and subrenal abdominal aortic aneurysm. However, for lesions that are adjacent to or involve the main branch vessels that need to be preserved, for example, type A dissection involving the ascending aorta and aortic arch, or abdominal aortic aneurysm involving the renal artery and important branches thereof, the conventional method at present is to replace the pathological artery with an artificial blood vessel by means of open surgery. During open surgery, the aortic blood flow needs to be completely blocked for a certain period of time and to a certain extent, which requires the close cooperation of surgery, anesthesia and extracorporeal circulation with the operating room. Currently, only a few hospitals have the ability to perform aortic surgery. In addition, due to the long-time extracorporeal circulation, myocardial blockage and body temperature fluctuations during aortic surgery, the postoperative mortality of aortic surgery and the incidence of other systemic complications are significantly higher than other cardiac surgeries, especially for the surgeries involving the aortic arch. Type A dissection account for 80% of the patients having dissection, and due to the difficult open surgery and the high incidence of postoperative complications, the open surgery is difficult to be deployed widely. New technologies have always been proposed and applied to extend the scope of use of minimally invasive endovascular aortic repair in recent years, for example, covered stents with branches, fenestration technology, chimney grafts, periscope grafts, and bypassing by hybrid surgery to preserve the blood supply of important branch vessels.

It is very difficult to guide the branches of an integrally formed stent with branches to in branch blood vessels and release them therein, and only single-branch stents are successfully used in minimally invasive surgery in clinic at present. The aortic arch has three important branches, and the distances therebetween and positions thereof are greatly different for different individuals. Multi-branch stents have been reported for clinical use in open surgery. However, it is very difficult to guide a stent with three branches into the corresponding branch vessels at the same time even in open surgery. The fenestration technology is to form a hole in a covering membrane of the covered stent at a position corresponding to an opening of a branch vessel, to maintain the blood supply in the branch vessel. When a fenestrated stent is positioned, it only requires to align the hole with the branch vessel as much as possible, and the operation is relatively simple. Therefore, the fenestration technology can be applied when the blood flow in multiple branches is preserved, however, it may readily cause type I endoleak. The chimney graft is similar to the periscope graft, and both of them have a small covered stent placed side by side outside the covered stent, for connecting a branch vessel to the aorta. This technology is currently mostly used to preserve a branch vessel. However, the covered branch stent is likely to be blocked by the compression of the aortic stent. In addition, because the covered stents are placed side by side, type I endoleak also tends to occur. Therefore, there is an urgent desire in clinic to develop a covered stent that is more suitable for branch vessels.

### SUMMARY

In view of this, the present invention provides a segmented covered stent suitable for a branch vessel. The following specific technical solution is adopted.

A segmented covered stent is provided, which includes a covering membrane and a support frame fixed to the covering membrane. The support frame includes an annular structure and a spiral structure, the annular structure is an annular structure formed by a plurality of first wave-shaped units connected end to end; and the spiral structure is a tubular structure formed by a plurality of second wave-shaped units connected end to end and arranged in a continuous spiral manner, and an overall extension direction of the spiral structure is parallel to the support frame.

Preferably, the annular structure includes a first annular structure and a second annular structure.

The support frame includes a proximal annular support frame, a middle spiral support frame, and a distal annular support frame in sequence from a proximal end to a distal end, wherein the proximal annular support frame incudes a plurality of first annular structures parallel to each other, the middle spiral support frame has a spiral structure, and the distal annular support frame includes a plurality of second annular structures parallel to each other.

Preferably, each of the first wave-shaped units includes a first peak, a first bar, and a first valley, with the first bar connecting the first peak to the first valley, circumferentially adjacent first bars are connected at a position adjacent to a proximal end to form a first peak, and circumferentially adjacent first bars are connected at a position adjacent to a distal end to form a first valley; and wherein each of the second wave-shaped units includes a second peak, a second bar, and a second valley, with the second bar connecting the second peak to the second valley, circumferentially adjacent second bars are connected at a position adjacent to a proximal end to form the second peak, and circumferentially adjacent second bars are connected at a position adjacent to a distal end to form the second valley.

Preferably, the first peak and the first valley of axially adjacent two loops of first wave-shaped units are located on a same axial axis.

Preferably, the first annular structure includes a first start bar, a first end bar, and a plurality of first wave-shaped units connected between the first start bar and the first end bar, wherein the first start bar is the first one of the first bars in a loop of the first annular structure, and the first end bar is the last one of the first bars in the loop of the first annular structure; and wherein the first start bar and the first end bar are connected by winding, welding, or being fixed by a steel sleeve.

Preferably, the first end bar of the first annular structure includes a first end extension section, wherein the first end extension section extends to a start position of an adjacent first annular structure, and the first end extension section forms the first start bar of the adjacent first annular structure, to thereby connect the two adjacent first annular structures.

Preferably, the second annular structure includes a second start bar, a second end bar, and a plurality of first wave-shaped units connected between the second start bar and the second end bar, wherein the second start bar is the first one of the first bars in a loop of the second annular structure, and the second end bar is the last one of the first bars in the loop of the second annular structure; and wherein the second start bar and the second end bar are connected by winding, welding, or being fixed by a steel sleeve.

Preferably, the second end bar of the second annular structure includes a second end extension section, wherein the second end extension section extends to a start position of an adjacent second annular structure, and the second end extension section forms the second start bar of the adjacent second annular structure, to thereby connect the two adjacent second annular structures.

Preferably, the middle spiral support frame includes a spiral start bar, a spiral end bar, and a spiral portion connected between the spiral start bar and the spiral end bar, wherein the spiral start bar is the first one of the second bars of the middle spiral support frame, and the spiral end bar is the last one of the second bars of the middle spiral support frame in a spiral extension direction.

Preferably, a proximal end and/or a distal end of the middle spiral support frame is/are connected to the annular structure by winding, welding or being fixed by a steel sleeve.

Preferably, the spiral start bar includes a spiral start extension section, wherein the spiral start extension section is connected to one of the first bars of the first annular structure, to connect the proximal annular support frame to the middle spiral support frame.

Preferably, the expression that "the spiral start extension section is connected to one of the first bars of the first annular structure" includes the spiral start extension section being connected to one of the first bars of the first annular structure closest to the middle spiral support frame.

Preferably, the spiral end bar includes a spiral end extension section, wherein the spiral end extension section is connected to one of the first bars of the second annular structure, to connect the middle spiral support frame to the distal annular support frame.

Preferably, the expression that "the spiral end extension section is connected to one of the first bars of the second annular structure" includes the spiral end extension section being connected to one of the first bars of the second annular structure closest to the middle spiral support frame.

Preferably, the second peaks of axially adjacent two second wave-shaped units are located on a same axial axis.

Preferably, the middle spiral support frame extends from the proximal end to the distal end, the middle spiral support frame includes a spiral portion formed by a plurality of spiral units, and each spiral unit has a spiral angle of 1° to 75°.

Preferably, the support frame is formed by a woven continuous wire.

Preferably, the continuous wire is a single-strand metal wire or a composite wire composed of a plurality of metal wires.

Preferably, the proximal annular support frame has a strength greater than a strength of the middle spiral support frame.

Preferably, a wire diameter of the metal wire used in the proximal annular support frame is larger than wire diameters of the metal wires used in the middle spiral support frame and in the distal annular support frame.

Preferably, a rigidity of the metal wire material used in the proximal annular support frame is greater than a rigidity of the metal wire material used in the middle spiral support frame.

Preferably, the support frame has a straight tube structure with consistent diameter or a conical tube structure with inconsistent diameters.

Preferably, the distal annular support frame includes a transitional second annular structure, wherein the second annular structure is provided at a side of the transitional second annular structure away from the proximal annular support frame; the transitional second annular structure has a flared with a diameter thereof gradually increasing in a direction from the proximal end to the distal end.

Preferably, the covering membrane includes an inner covering membrane and an outer covering membrane, wherein the support frame is arranged between the inner covering membrane and the outer covering membrane, the inner covering membrane is located inside the support frame, and the outer covering membrane is located outside the support frame.

Preferably, the segmented covered stent further includes at least one marker member.

The present invention also provides a method for preparing a segmented covered stent, which includes:
wrapping an inner covering membrane around a covering membrane applying mold rod;
mounting a support frame around a periphery of the inner covering membrane, wherein the support frame is in close contact with the inner covering membrane; and
wrapping an outer covering membrane around the support frame.

The present invention has the advantages as follows. The annular structure in the segmented covered stent provided in the present invention provides a good radial support ability to reduce the compressive force subjected to, the spiral structure has good flexibility, to adapt to the shape change for various curved and arch-shaped branch vessels, and the annular structure and the spiral structure are combined to adapt to different requirements of different branch vessels regarding the flexibility of the covered stent.

### BRIEF DESCRIPTION OF THE DRAWINGS

FIG. 1 is a schematic structural view of a segmented covered stent according to the present invention.
FIG. 2 is a schematic structural view of a first wave-shaped unit in a segmented covered stent according to the present invention.
FIG. 3 is a schematic structural view of a second wave-shaped unit in a segmented covered stent according to the present invention.
FIG. 4 is a schematic structural view of a support frame in a segmented covered stent according to the present invention.
FIG. 5 is a schematic structural view showing annular structures connected by a steel sleeve in a segmented covered stent according to the present invention.
FIG. 6 is a schematic structural view showing the interconnections in a support frame in a segmented covered stent according to the present invention.
FIG. 7 is a schematic structural view of a spiral start extension section connected and fixed to a first bar by a steel sleeve in a segmented covered stent according to the present invention.
FIG. 8 is a schematic structural view of a spiral start extension section connected to a first start bar in a segmented covered stent according to the present invention.
FIG. 9 is a schematic structural view of a segmented covered stent according to the present invention, showing a proximal annular support frame with a diameter greater than the diameter of a distal annular support frame.
FIG. 10 is a schematic structural view of a segmented covered stent according to the present invention, wherein a support frame thereof has a consistent-diameter straight tubular structure.
FIG. 11 is a schematic structural view of a segmented covered stent according to the present invention, when used in combination with an aortic stent.
FIG. 12 is a schematic structural view of another segmented covered stent according to the present invention, when used in combination with an aortic stent.
FIG. 13 is a schematic structural view of a segmented covered stent including an inner covering membrane and an outer covering membrane according to the present invention.
FIG. 14 is a flow chart of a method for preparing a segmented covered stent according to the present invention.
FIG. 15 is a schematic structural view of a segmented covered stent prepared according to the present invention.

### DESCRIPTION OF THE EMBODIMENTS

Preferred embodiments of the present invention will be described below. It should be noted that those skilled in the art can make various modifications and variations to the embodiments without departing from the spirit and scope of the present invention, and these modifications and variations should fall into the scope claimed by the present invention.

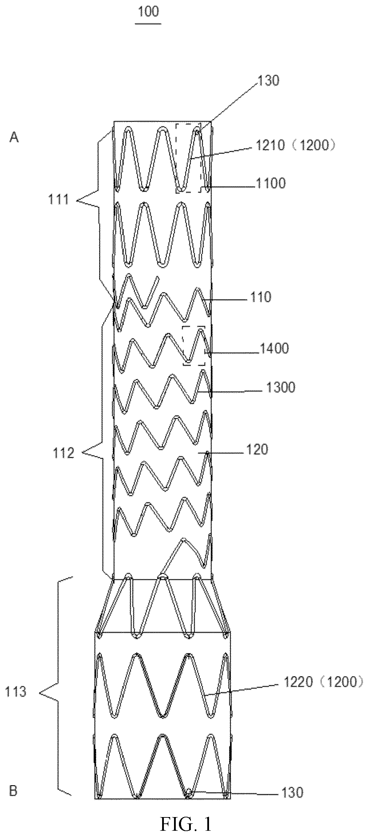

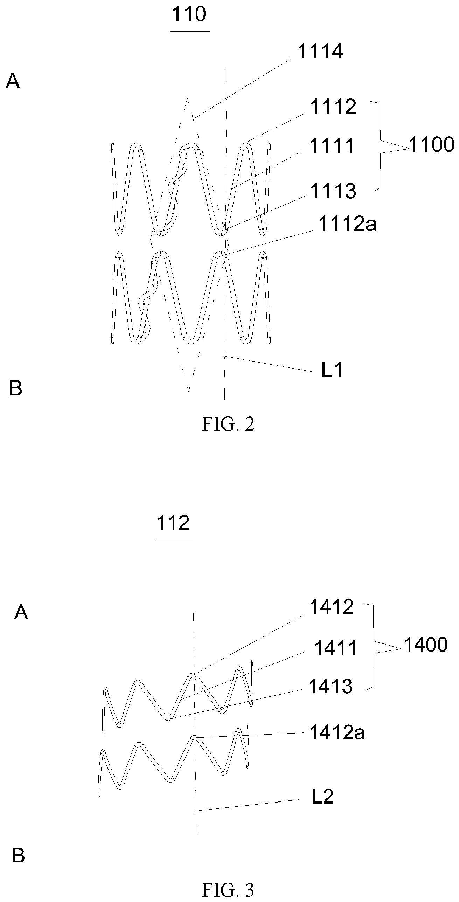

Referring to Fig. 1, the present invention provides a segmented covered stent 100. The segmented covered stent 100 includes a covering membrane 120 and a support frame 110 fixed to the covering membrane 120. The support frame 110 includes annular structures 1200 and a spiral structure 1300. The annular structure 1200 is an annular structure formed by a plurality of first wave-shaped units 1100 (see Fig. 2) connected end to end. A gap is provided between two adjacent annular structures 1200. The spiral structure 1300 is a tubular structure formed by a plurality of second wave-shaped units 1400 (see Fig. 3) connected end to end and arranged in a continuous spiral manner. The general extension direction of the spiral structure 1300 is parallel to the support frame 110. The annular structures 1200 provide a good radial support force to reduce the compression from other stents, The spiral structure 1300 has good flexibility to adapt to shape change for various curved and arch-shaped branch vessels. The annular structure 1200 and the spiral structure 1300 are combined to adapt to different requirements of different branch vessels regarding the flexibility of the covered stent.

In a further embodiment, the annular structure 1200 includes a first annular structure 1210 and a second annular structure 1220. The support frame 110 includes, from a proximal end A to a distal end B, a proximal annular support frame 111, a middle spiral support frame 112 and a distal annular support frame 113 in sequence. The proximal annular support frame 111 includes a plurality of first annular structures 1210 parallel to each other. The middle spiral support frame 112 has a spiral structure 1300. The distal annular support frame 113 includes a plurality of second annular structures 1220 parallel to each other. In the embodiment, the middle spiral support frame adopts a spiral structure 1300, having very good flexibility to adapt to shape change for various curved and arch-shaped branch vessels; and the proximal annular support frame 111 and the distal annular support frame 113 has annular structures 1200, so that the entire support frame 110 provides a good radial support force at two ends, to stabilize the support frame 110.

Referring to Fig. 2, in a further embodiment, in the proximal or distal annular structure, each of the first wave-shaped units 1100 includes a first peak 1112, a first bar 1111, and a first valley 1113, wherein the first bar 1111 connects the first peak 1112 to the first valley 1113. The circumferentially adjacent first bars 1111 are connected at a position close to a proximal end A to form the first peak 1112, and the circumferentially adjacent first bars 1111 are connected at a position close to a distal end B to form the first valley 1113. It can be understood that the circumferentially adjacent first peaks 1112may be located in the same level or in different levels, or may be arranged in such a manner that high peaks and low peaks are arranged alternately. Similarly, the circumferentially adjacent first valleys 1113 may be located in the same level or in different levels, or may be arranged in such a manner that high valleys and low valleys are arranged alternately. It can be understood that the first wave-shaped units 1100 form one or more of U-shaped waves, V-shaped waves, and S-shaped waves. It can be understood that the first wave-shaped units 1100 in the first annular structure 1210 and the second annular structure 1220 may be the same, or adaptively adjusted in size.

In a further embodiment, the length of the first bar 1111 in the first wave-shaped unit 1100 is 3-10 mm. The angle formed by the extension lines of two adjacent first bars 1111 is 30-150 degrees. The height of the first wave-shaped unit is 3-10 mm. In a further embodiment, within the proximal or distal annular structure, the first peak 1112 and first valley 1113 of axially adjacent two rings of first wave-shaped units 1100 are located on the same axial axis. For example, the peak 1112a and the valley 1113 in Fig. 2 are located on the same axial axis L1, wherein the axial axis L1 is parallel to the support frame. As shown in Fig. 2, the first peak 1112a and the first valley 1113 are located on the same axial axis L1, which means that the peaks of one annular structure are respectively aligned with the valleys of the axially adjacent annular structure are adjacent (with the shortest distance). That is, the distance between the first peak 1112a and the first valley 1113 is the shortest, so that diamond-like shaped unit structures 1114 can be formed among the axially adjacent first wave-shaped units 1100, thereby increasing the radial support abilities of the proximal annular support frame 111 and the distal annular support frame 113 formed by the first wave-shaped units 1100.

Referring to Fig. 3, in the middle spiral structure, each of the second wave-shaped units 1400 includes a second peak 1412, a second bar 1411, and a second valley 1413, wherein the second bar 1411 connects the second peak 1412 to the second valley 1413. The circumferentially adjacent second bars 1411 are connected at a position close to a proximal end A to form the second peak 1412, and the circumferentially adjacent second bars 1411 are connected at a position close to a distal end B to form the second valley 1413. It can be understood that the circumferentially adjacent second peaks 1412may be located in the same level or in different levels, or may be arranged in such a manner that high peaks and low peaks are arranged alternately. Similarly, the circumferentially adjacent second valleys 1413 may be located in the same level or in different levels, or may be arranged in such a manner that high valleys and low valleys are arranged alternately. It can be understood that the second wave-shaped units 1400 form one or more of U-shaped waves, a V-shaped waves, and an S-shaped waves.

In a further embodiment, the length of the second bar 1411 in the second wave-shaped unit 1400 is 3-10 mm. The angle formed by the extension lines of two adjacent second bars 1411 is 30-150 degrees. The height of the second wave-shaped unit is 3-10 mm.

In a further embodiment, within the middle spiral structure, the second peaks 1412 of axially adjacent two second wave-shaped units 1400 are located on the same axial axis. For example, the second peak 1412 and the axially adjacent second peak 1412a in Fig. 3 are located on the same axial axis L2. It can be understood that the direction of the axial axis L2 is parallel to the support frame. That is, the second peaks 1412 of axially adjacent two second wave-shaped units 1400 in the middle spiral support frame 112 are aligned. For example, the second peak 1412 and the axially adjacent second peak 1412a in Fig. 3 are aligned, so that more space are provided between the second peak 1412 and the axially adjacent second peak 1412a for moving towards each other, thus ensuring the flexibility of the spiral structure 1300.

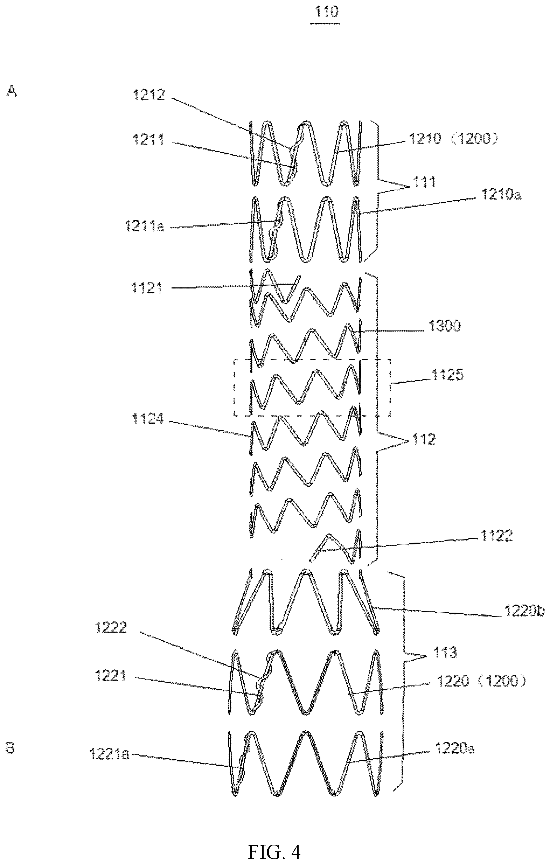

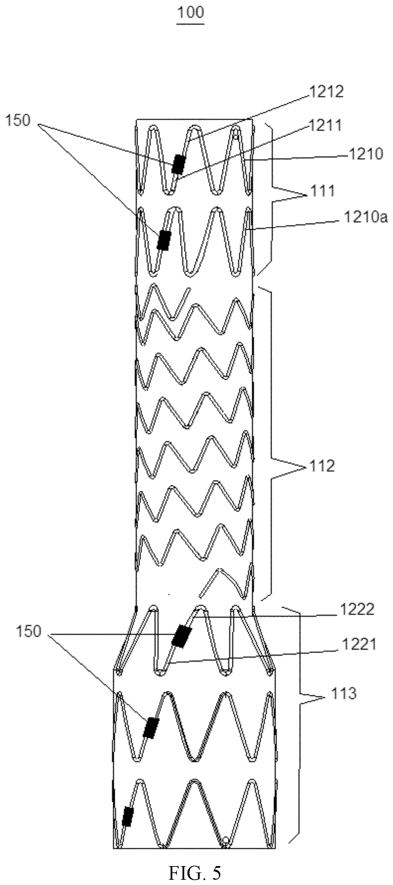

Referring to Fig. 4, in a further embodiment, the first annular structure 1210 includes a first start bar 1211, a first end bar 1212, and a plurality of first wave-shaped units connected between the first start bar 1211 and the first end bar 1212. The first start bar 1211 is the first one of the first bars in the loop of the first annular structure, and the first end bar 1212 is the last one of the first bars in the loop of the first annular structure. The first start bar 1211 and the first end bar 1212 are connected by winding, welding, or being fixed by a steel sleeve. Fig. 4 shows the connection by winding the first end bar 1212 on the first start bar 1211. Referring to Fig. 5, the first end bar 1212 and the first start bar 1211 are fixed by a steel sleeve 150.

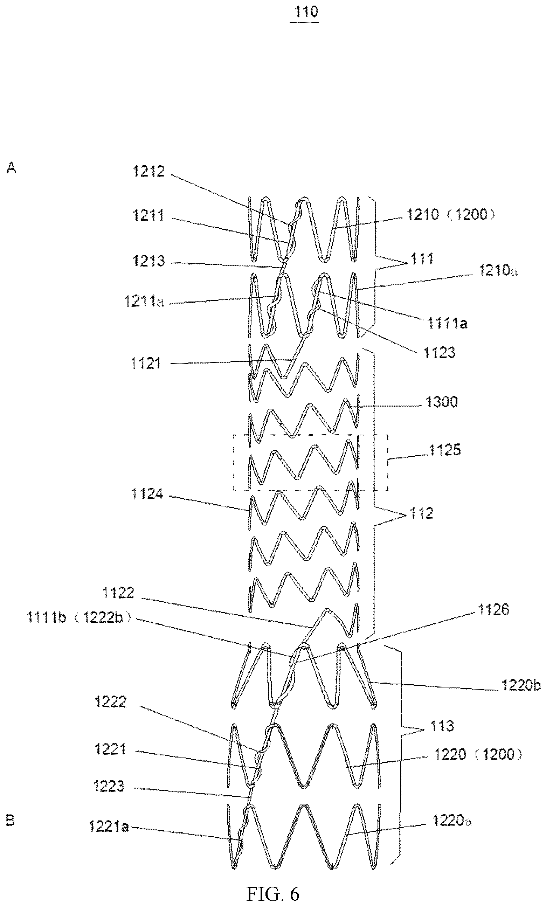

Referring to Fig. 6, in a further embodiment, the first end bar 1212 of the first annular structure 1210 includes a first end extension section 1213, which extends to a start position of an adjacent first annular structure 1210a, and forms a first start bar 1211a of the adjacent first annular structure 1210a, to connect the first annular structure 1210 to the adjacent first annular structure 1210a, so that the connection between the first annular structures 1210 is more firm and stable.

Referring to Fig. 4, in a further embodiment, the second annular structure 1220 includes a second start bar 1221, a second end bar 1222, and a plurality of first wave-shaped units connected between the second start bar 1221 and the second end bar 1222. The second start bar 1221 is the first one of the first bars in the loop of the second annular structure 1220, and the second end bar 1222 is the last one of the first bars in the loop of the second annular structure 1220. The second start bar 1221 and the second end bar 1222 are connected by winding, welding, or being fixed by a steel sleeve. Fig. 4 shows the connection by winding the second end bar 1222 on the second start bar 1221. In Fig. 5, the second end bar 1222 and the second start bar 1221 are fixed by a steel sleeve 150.

Referring to Fig. 6, in a further embodiment, the second end bar 1222 of the second annular structure 1220 includes a second end extension section 1223, which extends to a start position of an adjacent second annular structure 1220a, and forms a second start bar 1221a of the adjacent second annular structure 1220a, to connect the second annular structure 1220 to the adjacent second annular structure 1220a, so that the connection between the second annular structures 1220 is more firm and stable.

Referring to Fig. 4, in a further embodiment, the middle spiral support frame 112 includes a spiral start bar 1121, a spiral end bar 1122, and a spiral portion 1124 connected between the spiral start bar 1121 and the spiral end bar 1122. The spiral start bar 1121 is the first one of the second bars of the middle spiral support frame 112, and the spiral end bar 1122 is the last one of the second bars of the middle spiral support frame 112 along the spiral extension direction. The spiral start bar 1121 is arranged closer to the proximal annular support frame 111 than the spiral end bar 1122. The spiral portion 1124 is formed by a plurality of spiral units 1125 connected one another, and extends spirally from a proximal end A to a distal end B. The general spiral direction of the middle spiral support frame 112 refers to the direction from the proximal end A to the distal end B.

In a further embodiment, the proximal end and/or the distal end of the middle spiral support frame 112 is/are connected to the annular structure 1200 by winding, welding or being fixed by a steel sleeve.

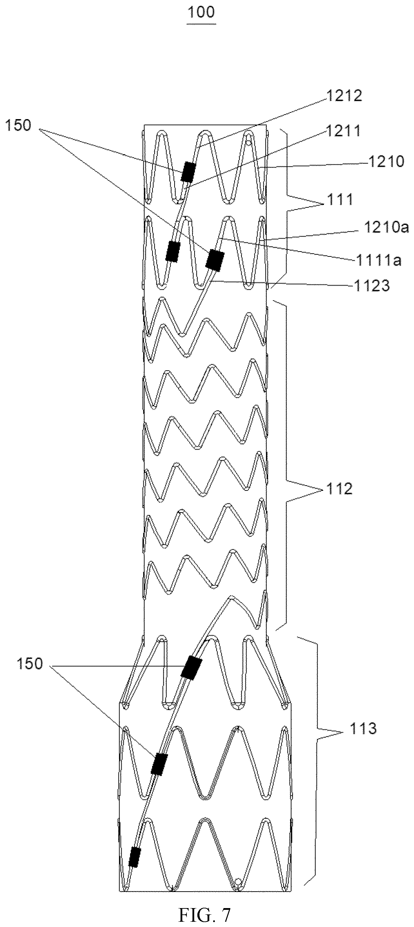

Referring to Fig. 6, in a further embodiment, the spiral start bar 1121 includes a spiral start extension section 1123, which is connected to one of the first bars 1111a in the first annular structure 1210a in the proximal annular support frame 111, to connect the proximal annular support 111 to the middle spiral support frame 112. The spiral start extension section 1123 is connected to one of the first bars 1210a in the first annular structure 1210a by winding, welding, or being fixed by a steel sleeve. In Fig. 6, the spiral start extension section 1123 is wound on the first bar 1111a. Referring to Fig. 7, the spiral start extension section 1123 is connected and fixed to the first bar 1111a by a steel sleeve 150.

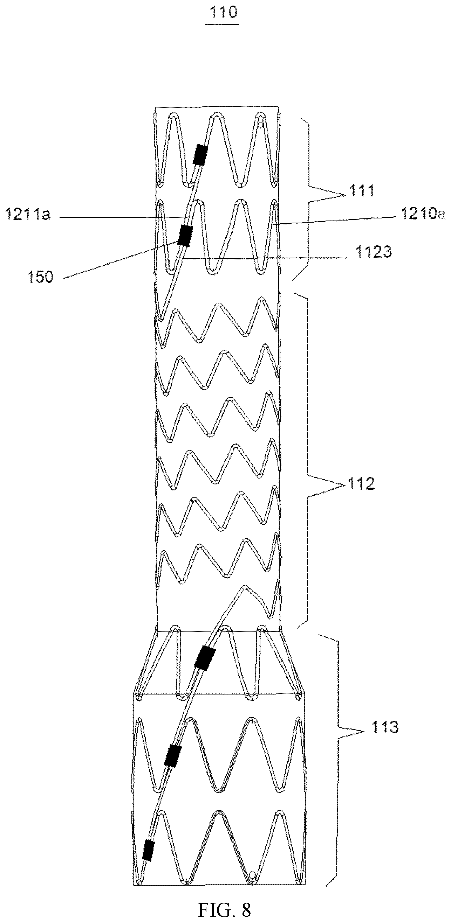

It can be understood that referring to Fig. 8, the expression that "the spiral start extension section 1123 is connected to one of the first bars 1111a in the first annular structure 1210a in the proximal annular support frame 111" includes the case that the spiral start extension section 1123 is connected to the first start bar 1211a in the first annular structure 1210a in the proximal annular support frame 111. In FIG. 8, the connection is achieved by a steel sleeve 150.

Referring to Fig. 6, in a further embodiment, the expression that "the spiral start extension section 1123 is connected to one of the first bars 1111a in the first annular structure 1210a in the proximal annular support frame 111" includes the case that the spiral start extension section 1123 is connected to the first bar 1111a in the first annular structure 1210a which is closest to the middle spiral support frame 112. The first annular structure 1210a closest to the middle spiral support frame 112 is the first annular structure 1210a as shown in Fig. 6.

It can be understood that the spiral start extension section 1123 may be connected to the first bar in the first annular structure 1210 at any position in the proximal annular support frame 111. That is, the spiral start bar 1121 of the middle spiral support frame 112 may extend into the proximal annular support frame 111, thereby further improving the stability of the overall structure of the stent.

The spiral end bar 1122 includes a spiral end extension section 1126, which is connected to one of the first bars 1111b in the second annular structure 1220b, to connect the middle spiral support frame 112 to the distal annular support frame 113. The spiral end extension section 1126 is connected to one of the first bars 1111b in the second annular structure 1220b by winding, welding, or being fixed by a steel sleeve. In Fig. 6, the spiral bar 1111b is wound on the spiral end extension section 1126.

It can be understood that the expression that "the spiral end extension section 1126 is connected to one of the first bars 1111b in the second annular structure 1220b" includes the case that the spiral end extension section 1126 is connected to the second end bar 1222b in the second annular structure 1220.

In a further embodiment, the expression that "the spiral end extension section 1126 is connected to one of the first bars 1111b in the second annular structure 1220b" includes the case that the spiral end extension section 1126 is connected to one of the first bars 1111b in the second annular structure 1220b closest to the middle spiral support frame 112. The second annular structure 1220b closest to the middle spiral support frame 112 is the second annular structure 1220b as shown in Fig. 6.

It can be understood that the spiral end extension section 1126 may be connected to the first bar in the second annular structure 1220 at any position in the distal annular support frame 113. That is, the spiral end bar 1122 of the middle spiral support frame 112 may extend into the distal annular support frame 113, thereby further improving the stability of the overall structure of the stent.

The proximal and distal ends of the middle spiral support frame 112 are respectively connected to the annular structures, such that the support frame 110 is connected together as a whole. With interaction between various segments, the support frame 110 has a higher stability in radial and axial directions.

Referring to Fig. 4, in a further embodiment, the distal annular support frame 113 includes a transitional second annular structure 1220b. The second annular structure 1220 is provided at one side of the transitional second annular structure 1220b away from the proximal annular support frame 111. The transitional second annular structure 1220b has a flared shape, with a diameter thereof gradually increases in a direction from a proximal end to a distal end. When the distal annular support frame 113 enters a branch vessel, the larger diameter can increase the conformation to the vessel wall, reducing the occurrence of endoleak. The transitional second annular structure 1220b can well connect the middle spiral support frame 112 to the second annular structure 1220 in the distal annular support frame 113, so as to further increase the overall stability of the segmented covered stent 100.

It can be understood that the transitional second annular structure 1220b has a smallest diameter at a position close to a proximal end A, and the smallest diameter is equal to that of the middle spiral support frame 112. The transitional second annular structure 1220b has a largest diameter at a position close to a distal end B, and the largest diameter is equal to that of the second annular structure 1220, so as to well connect the middle spiral support frame 112 to the second annular structure 1220 in the distal annular support frame 113.

In a further embodiment, the proximal annular support frame 111 has an axial length of 5-50 mm.

In a further embodiment, the middle spiral support frame 112 extends from the proximal end A to the distal end B. The middle spiral support frame 112 includes the spiral portion 1124 having a plurality of spiral units 1125. Each spiral unit 1125 has a spiral angle of 1-75°. Preferably, the spiral unit 1125 has a spiral angle of 5-45°. Within the above spiral angle range, the middle spiral support frame 112 has a very good flexibility, and can be bent freely, to adapt to various curved or arch-shaped blood vessels.

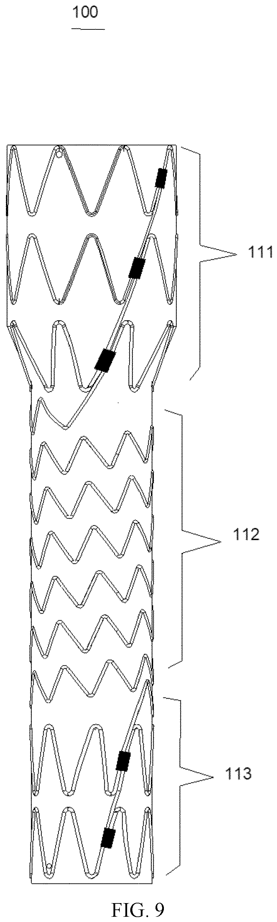

Referring to Fig. 9, it can be understood that in other embodiments, the proximal annular support frame 111 may have a diameter larger than that of the distal annular support frame 113, applicable for a blood vessel with a larger diameter at the proximal end A and a smaller diameter at the distal end B.

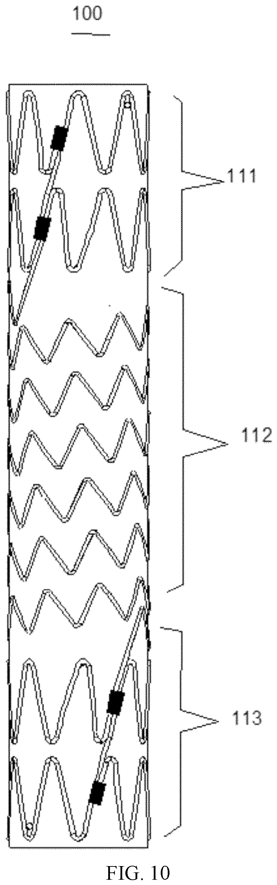

Referring to Fig. 10, it can be understood that in other embodiments, the proximal annular support frame 111 may have a diameter equaling to that of the distal annular support frame 113, as such, the entire support frame 111 is a straight tubular stent extending with consistent diameter.

In a further embodiment, the support frame 110 is formed by a woven continuous wire. The continuous wire may be a single-strand wire, or a multi-strand wire. The material of the wire may be selected from metal materials or polymer materials, wherein the metal material can be selected from stainless steel, cobalt alloys, tantalum, nickel titanium alloys, or other biocompatible metals; and preferably shape memory alloys. The multi-strand wire can be formed by twisting or weaving multiple strands of wires, wherein the material of the multiple strands of wires may be the same or different.

In a further embodiment, the continuous wire is a single metal wire or a composite wire composed of a plurality of metal wires, for example, stainless steel cobalt alloys, tantalum, nickel titanium alloys, or other biocompatible metals, preferably shape memory alloys, and more preferably nickel titanium alloys. The woven support frame 110 formed by a continuous metal wire has an increased stability.

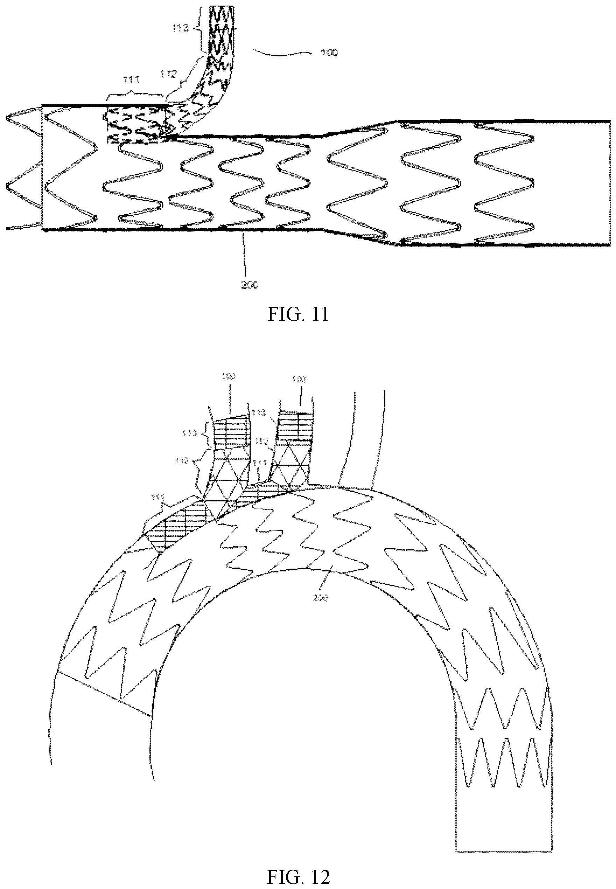

Referring to Fig. 11, when the segmented covered stent 100 is used in combination with an aortic stent 200, the proximal support frame 111 will be compressed by the aortic stent 200, and thus a larger radial support force is required in this case. Moreover, as shown in FIG. 12 (where the particular structures of the annular support frame and the spiral support frame are not specifically shown), several branch stents need to be used simultaneously at some vessels with multiple branches. In this case, the proximal end of the branch stent will further be compressed by the adjacent branch stents. Therefore, in a further embodiment, the strength of the proximal annular support frame 111 is greater than the strength of the middle spiral support frame 112.For this purpose, the rigidity of the metal wire material used in the proximal annular support frame 111 is greater than the rigidity of the metal wire material used in the middle spiral support frame 112, or the wire diameter of the metal wire used in the proximal annular support frame 111 is larger than the wire diameter of the metal wire used in the middle spiral support frame 112, to ensure that the proximal annular support frame 111 has a good radial support ability and can be firmly connected with other branch stents in the blood vessel. Preferably, the wire diameter of the metal wire used in the proximal annular support frame 111 is 0.3 mm.

In a further embodiment, the wire diameter of the metal wire used in the middle spiral support frame 112 is smaller than the wire diameter of the distal annular support frame 112, to ensure that the middle spiral support frame 112 has good flexibility. Preferably, the wire diameter of the metal wire used in the middle spiral support frame 112 is 0.25 mm.

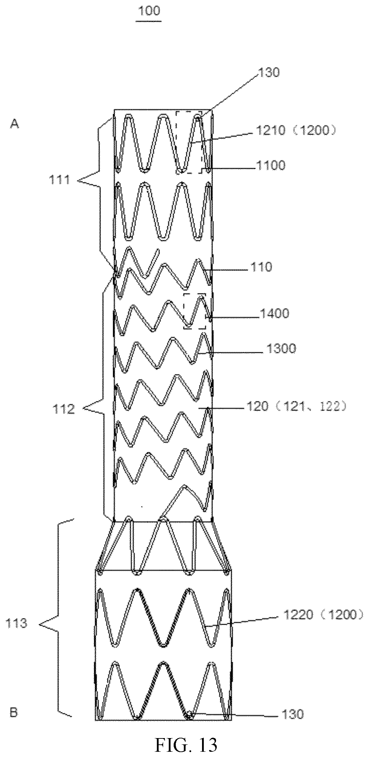

Referring to Fig. 13 again, in a further embodiment, the covering membrane 120 may include an inner covering membrane 121 and an outer covering membrane 122. The support frame 110 is arranged between the inner covering membrane 121 and the outer covering membrane 122, with the inner covering membrane 121 located inside the support frame 110, and the outer covering membrane 122 located outside the support frame 110. The materials of the inner covering membrane 121 and the outer covering membrane 122 may be independently selected from polymer materials with excellent biocompatibility, for example, expanded polytetrafluoroethylene, PET, polyesters, polyurethane, silicone, ultra-high molecular weight polyethylene or other suitable materials. Preferably, both the inner covering membrane 121 and the outer covering membrane 122 are made of expanded polytetrafluoroethylene material. Expanded polytetrafluoroethylene can well prevent fluid penetration and increase the radial support ability of the segmented covered stent 100.

In a further embodiment, the inner covering membrane 121 and the outer covering membrane 122 are each formed as one piece.

In a further embodiment, the inner covering membrane 121 and the outer covering membrane 122 are together formed as one piece, to make the segmented covered stent firmer. It can be understood that the inner covering membrane 121 and the outer covering membrane 122 can be formed as one piece by thermal bonding.

In a further embodiment, the segmented covered stent100 may further include at least one marker member 130, which may be arranged annularly or along a track of figure 8. The marker member may be fixed to the proximal and distal ends of the support frame 110 by suturing or welding. The material of the marker member can be selected from gold, platinum, platinum-tungsten, palladium, platinum-iridium, rhodium, tantalum, or alloys or composites of these metals. Preferably, two marker members 130 are provided and the two marker members 130 are symmetrically provided on the support frame 110. Further, the two marker members 130 are respectively located on a first peak of the proximal annular support frame 111 and a first valley of the distal annular support frame 112, to ensure that the segmented covered stent 100 as a whole can be effectively monitored.

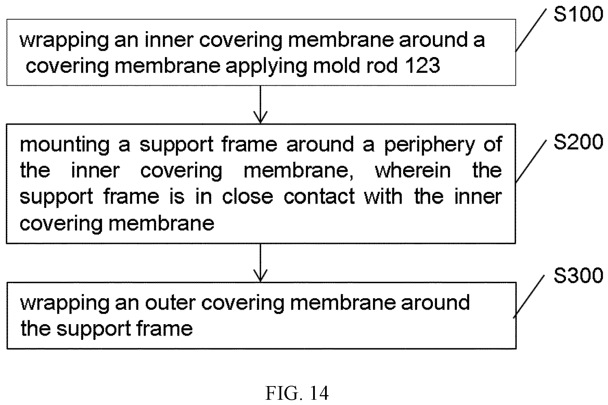

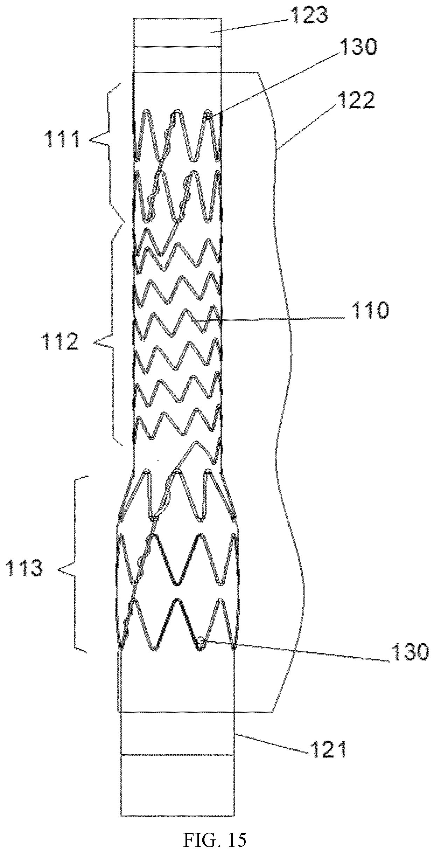

Referring to FIGs. 14 and 15, the present invention also provides a method for preparing a segmented covered stent. The method for preparing the segmented covered stent 100 includes Step S100, Step S200 and Step S300. The steps are specifically described below.

Step S100: wrapping an inner covering membrane 121 around a covering membrane applying mold rod 123.

Step S200: mounting a support frame 110 around a periphery of the inner covering membrane 121, wherein the support frame 121 is in close contact with the inner covering membrane 121; and the support frame 110 may be anyone of the support frames according to the above embodiments.

Step S300: wrapping an outer covering membrane around the support frame.

The above embodiments are only several implementations of the present application, which are described specifically and in detail, without limitation to the scope claimed by the present application. It should be noted that those skilled in the art can make various modifications and variations to the embodiments without departing from the spirit and scope of the present application, and these modifications and variations should fall into the scope claimed by the appended claims.

## Claims

1. A segmented covered stent, comprising a covering membrane and a support frame fixed to the covering membrane, wherein the support frame comprises an annular structure and a spiral structure, and
wherein the annular structure is by a plurality of first wave-shaped units connected end to end; and the spiral structure is a tubular structure formed by a plurality of second wave-shaped units connected end to end and arranged in a continuous spiral manner, and an overall extension direction of the spiral structure is parallel to the support frame.

2. The segmented covered stent according to claim 1, wherein the annular structure comprises a first annular structure and a second annular structure; and
the support frame comprises, a proximal annular support frame, a middle spiral support frame, and a distal annular support frame in sequence from a proximal end to a distal end, wherein the proximal annular support frame comprises a plurality of first annular structures parallel to each other, the middle spiral support frame has a spiral structure, and the distal annular support frame comprises a plurality of second annular structures parallel to each other.

3. The segmented covered stent according to claim 2, wherein each of the first wave-shaped units comprises a first peak, a first bar, and a first valley, with the first bar connecting the first peak to the first valley, circumferentially adjacent first bars are connected at a position adjacent to a proximal end to form a first peak, and circumferentially adjacent first bars are connected at a position adjacent to a distal end to form a first valley; and wherein each of the second wave-shaped units comprises a second peak, a second bar, and a second valley, with the second bar connecting the second peak to the second valley, circumferentially adjacent second bars are connected at a position adjacent to a proximal end to form the second peak, and circumferentially adjacent second bars are connected at a position adjacent to a distal end to form the second valley.

4. The segmented covered stent according to claim 3, wherein the first peak and the first valley of axially adjacent first wave-shaped units are located on a same axial axis.

5. The segmented covered stent according to claim 3, wherein the first annular structure comprises a first start bar, a first end bar, and a plurality of first wave-shaped units connected between the first start bar and the first end bar, wherein the first start bar is the first one of the first bars in a loop of the first annular structure, and the first end bar is the last one of the first bars in the loop of the first annular structure; and wherein the first start bar and the first end bar are connected by winding, welding, or being fixed by a steel sleeve.

6. The segmented covered stent according to claim 5, wherein the first end bar of the first annular structure comprises a first end extension section, wherein the first end extension section extends to a start position of an adjacent first annular structure, and the first end extension section forms the first start bar of the adjacent first annular structure, to thereby connect the two adjacent first annular structures.

7. The segmented covered stent according to claim 5, wherein the second annular structure comprises a second start bar, a second end bar, and a plurality of first wave-shaped units connected between the second start bar and the second end bar, wherein the second start bar is the first one of the first bars in a loop of the second annular structure, and the second end bar is the last one of the first bars in the loop of the second annular structure; and wherein the second start bar and the second end bar are connected by winding, welding, or being fixed by a steel sleeve.

8. The segmented covered stent according to claim 7, wherein the second end bar of the second annular structure comprises a second end extension section, wherein the second end extension section extends to a start position of an adjacent second annular structure, and the second end extension section forms the second start bar of the adjacent second annular structure, to thereby connect the two adjacent second annular structures.

9. The segmented covered stent according to claim 7, wherein the middle spiral support frame comprises a spiral start bar, a spiral end bar, and a spiral portion connected between the spiral start bar and the spiral end bar, wherein the spiral start bar is the first one of the second bars of the middle spiral support frame, and the spiral end bar is the last one of the second bars of the middle spiral support frame in a spiral extension direction.

10. The segmented covered stent according to claim 9, wherein a proximal end and/or a distal end of the middle spiral support frame is/are connected to the annular structure by winding, welding or being fixed by a steel sleeve.

11. The segmented covered stent according to claim 10, wherein the spiral start bar comprises a spiral start extension section, wherein the spiral start extension section is connected to one of the first bars of the first annular structure, to connect the proximal annular support frame to the middle spiral support frame.

12. The segmented covered stent according to claim 11, wherein the expression that "the spiral start extension section is connected to one of the first bars of the first annular structure" comprises the spiral start extension section being connected to one of the first bars of the first annular structure closest to the middle spiral support frame.

13. The segmented covered stent according to claim 10, wherein the spiral end bar comprises a spiral end extension section, wherein the spiral end extension section is connected to one of the first bars of the second annular structure, to connect the middle spiral support frame to the distal annular support frame.

14. The segmented covered stent according to claim 13, wherein the expression that "the spiral end extension section is connected to one of the first bars of the second annular structure" comprises the spiral end extension section being connected to one of the first bars of the second annular structure closest to the middle spiral support frame.

15. The segmented covered stent according to claim 3, wherein the second peaks of axially adjacent two second wave-shaped units are located on a same axial axis.

16. The segmented covered stent according to claim 2, wherein the middle spiral support frame extends from the proximal end to the distal end, the middle spiral support frame comprises a spiral portion formed by a plurality of spiral units, and each spiral unit has a spiral angle of 1° to 75°.

17. The segmented covered stent according to claim 2, wherein the support frame is formed by a woven continuous wire.

18. The segmented covered stent according to claim 17, wherein the continuous wire is a single-strand metal wire or a composite wire composed of a plurality of metal wires.

19. The segmented covered stent according to claim 18, wherein the proximal annular support frame has a strength greater than a strength of the middle spiral support frame.

20. The segmented covered stent according to claim 19, wherein a wire diameter of the metal wire used in the proximal annular support frame is larger than wire diameters of the metal wires used in the middle spiral support frame and in the distal annular support frame.

21. The segmented covered stent according to claim 19, wherein a rigidity of the metal wire material used in the proximal annular support frame is greater than a rigidity of the metal wire material used in the middle spiral support frame.

22. The segmented covered stent according to claim 1, wherein the support frame has a straight tube structure with consistent diameter or a conical tube structure with inconsistent diameters.

23. The segmented covered stent according to claim 2, wherein the distal annular support frame comprises a transitional second annular structure, wherein the second annular structure is provided at a side of the transitional second annular structure away from the proximal annular support frame; the transitional second annular structure has a flared with a diameter thereof gradually increasing in a direction from the proximal end to the distal end.

24. The segmented covered stent according to claim 1, wherein the covering membrane comprises an inner covering membrane and an outer covering membrane, wherein the support frame is arranged between the inner covering membrane and the outer covering membrane, the inner covering membrane is located inside the support frame, and the outer covering membrane is located outside the support frame.

25. The segmented covered stent according to claim 2, wherein the segmented covered stent further comprises at least one marker member.

26. A method for preparing a segmented covered stent, comprising wrapping an inner covering membrane around a covering membrane applying mold rod;
mounting a support frame around a periphery of the inner covering membrane, wherein the support frame is in close contact with the inner covering membrane; and
wrapping an outer covering membrane around the support frame.
